# EUROPEAN PATENT APPLICATION

(11) **EP 2 962 717 A1**
(43) Date of publication of application: **06.01.2016**
(21) Application number: 15174363.0
(22) Date of filing: 29.06.2015
(51) Int. Cl.: A61M 25/02, A61M 27/00

(54) **DRAINAGE TUBE FOR TREATMENT OF BARTHOLIN´S DUCT ABSCESS**

(30) Priority: 30.06.2014 KR 20140081230
(71) Applicant: Woo Young Medical Co., Ltd., Chungcheongbuk-do 365-801 (KR)
(72) Inventor: LEE, Young Gyu, 135-280 Seoul (KR); PARK, Hyun Jeong, 361-735 Chungcheongbuk-do (KR)
(74) Representative: Mammel und Maser

(57) **Abstract**

A drainage tube including: a conduit member 10 which has a first end 12, a second end 14, and a drainage passage 16 disposed between the two ends, the first end being inserted into a diseased part of a Bartholin's duct abscess through an incision; a first wing member 20 which is provided on an outer circumference at a side of the first end and prevents the conduit member inserted into the diseased part from being separated; and a second wing member 30 which is provided on the outer circumference at a side of the second end and restricts the depth to which the conduit member is inserted into the diseased part, from the diseased part while the incision heals. The drainage tube may be removed after the incision heals and a drainage opening, which corresponds to the outer circumference of the conduit member is created.

## Description

### BACKGROUND OF THE INVENTION

### 1. Field of the Invention

The present invention relates to a drainage tube that is used to treat a Bartholin's duct abscess (also called a Bartholin's abscess) occurs in a female Bartholin's duct.

### 2. Description of the Related Art

A Bartholin's duct abscess is a disease that often occurs in females, and the Bartholin's duct abscess occurs when a Bartholin's duct increases in size due to an abscess (pustule) caused by inflammation and damage of the Bartholin's duct that drains mucus secreted by a Bartholin's gland located at a female introitus. When the Bartholin's duct is clogged, the Bartholin's duct increases in diameter up to 3 cm to 5 cm.

In general, in order to treat the Bartholin's duct abscess, marsupialization or resection is carried out. The marsupialization for the Bartholin's duct abscess refers to a surgical procedure that creates a drainage opening, which is an outlet of pus, so as to allow the pus to flow out from the Bartholin's duct abscess. The resection of the Bartholin's duct abscess is mainly carried out when the Bartholin's duct abscess recurs even though the marsupialization has been carried out, and refers to a surgical procedure that completely removes the Bartholin's duct abscess.

The marsupialization for treatment of the Bartholin's duct abscess is generally carried out in an operating theatre while a patient is under sedation anesthesia. However, in many cases, a surgical time is prolonged and the patient often awakens from the sedation anesthesia, and as a result, patient needs to undergo additional sedation anesthesia, which may put the patient's life in danger. In addition, in a case in which the patient moves during the surgical procedure, it is inevitably difficult to perform the surgical procedure. In addition, because the drainage opening, which has been created during the surgical procedure, is frequently clogged while the patient recuperates from the surgical procedure, the Bartholin's duct abscess often recurs. In a case in which the Bartholin's duct abscess recurs, it is necessary to perform the resection of the Bartholin's duct abscess which requires a long period of time for the surgical procedure and the recuperation.

### SUMMARY OF THE INVENTION

The present invention has been made in an effort to provide a drainage tube that is advantageous in terms of a surgical procedure for treatment of a Bartholin's duct abscess, and may reduce a burden on a patient.

An exemplary embodiment of the present invention provides a drainage tube including: a conduit member which has a first end, a second end, and a drainage passage disposed between the first end and the second end, the first end being inserted into a diseased part of a Bartholin's duct abscess through an incision created by incising the diseased part; a first wing member which protrudes from an outer circumference of the conduit member at a side of the first end and prevents the conduit member inserted into the diseased part from being separated; and a second wing member which protrudes from the outer circumference of the conduit member at a side of the second end and restricts the depth to which the conduit member is inserted into the diseased part.

According to the drainage tube according to the exemplary embodiment of the present invention, pus or the like may be smoothly drained from the diseased part while the incision (surgical site) heals, and the drainage tube may be removed from the surgical site after the incision heals and a drainage opening, which corresponds to the outer circumference of the conduit member, is created in the surgical site.

The first wing member and the second wing member may be provided in an outer circumferential direction of the conduit member and may have a rim-shaped structure.

At least the first wing member of the first and second wing members may be made of a material having predetermined elasticity.

The conduit member, the first wing member, and the second wing member may be formed integrally.

Another exemplary embodiment of the present invention provides a drainage tube including: a conduit member which has a first end, a second end, and a drainage passage disposed between the first end and the second end, the first end being inserted into a diseased part of a Bartholin's duct abscess through an incision created by incising the diseased part; and a wing member which protrudes from an outer circumference of the conduit member at a side of the first end and prevents the conduit member inserted into the diseased part from being separated.

Yet another exemplary embodiment of the present invention provides a drainage tube including: a conduit member which has a first end, a second end, and a drainage passage disposed between the first end and the second end, the first end being inserted into a diseased part of a Bartholin's duct abscess through an incision created by incising the diseased part; and a wing member which protrudes from an outer circumference of the conduit member at a side of the second end and restricts the depth to which the conduit member is inserted into the diseased part.

According to the exemplary embodiment of the present invention, since the surgical procedure (marsupialization) for treatment of the Bartholin's duct abscess may be conveniently and quickly carried out, the surgical procedure may be carried out through outpatient clinics while a patient is under local anesthesia. Further, in a case in which the surgical procedure is carried out in an operating theatre, it is possible to reduce the amount of time for sedation anesthesia, thereby greatly reducing a burden on the patient.

In addition, it is possible to prevent a drainage opening, which is created in the diseased part of the Bartholin's duct abscess during the surgical procedure, from being clogged (failure of the surgical procedure) during the recuperation process after performing the surgical procedure, thereby minimizing the occurrence of problems that the resection needs to be carried out because of the recurrence of the Bartholin's duct abscess.

### BRIEF DESCRIPTION OF THE DRAWINGS

FIG. 1 is a perspective view illustrating a drainage tube according to a first exemplary embodiment of the present invention.
FIG. 2 is a longitudinal cross-sectional view illustrating the drainage tube according to the first exemplary embodiment of the present invention.
FIG. 3 is a transverse cross-sectional view (a cross-sectional view taken along line A-A of FIG. 2) illustrating the drainage tube according to the first exemplary embodiment of the present invention.
FIGS. 4, 5 and 6 are a view illustrating a state in which the drainage tube according to the first exemplary embodiment of the present invention is used.
FIG. 7 is a transverse cross-sectional view illustrating a drainage tube according to a second exemplary embodiment of the present invention.
FIG. 8 is a front view illustrating a drainage tube according to a third exemplary embodiment of the present invention.

### DESCRIPTION OF MAIN REFERENCE NUMERALS

10: Conduit member
12: First end
14: Second end
16: Drainage passage
20: First wing member
30: Second wing member

### DESCRIPTION OF THE PREFERRED EMBODIMENTS

Hereinafter, exemplary embodiments of the present invention will be described with reference to the accompanying drawings.

A drainage tube according to the exemplary embodiments of the present invention is used for a surgical procedure (marsupialization) which creates a drainage opening that is an outlet through which pus and the like are drained from the abscess while a surgical site heals, and mucus from a Bartholin's gland is drained after the surgical site heals, at a diseased part of a Bartholin's duct abscess.

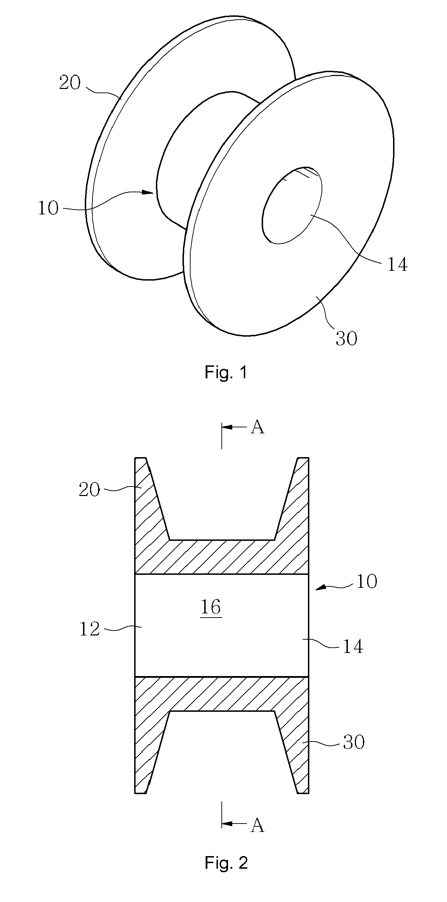

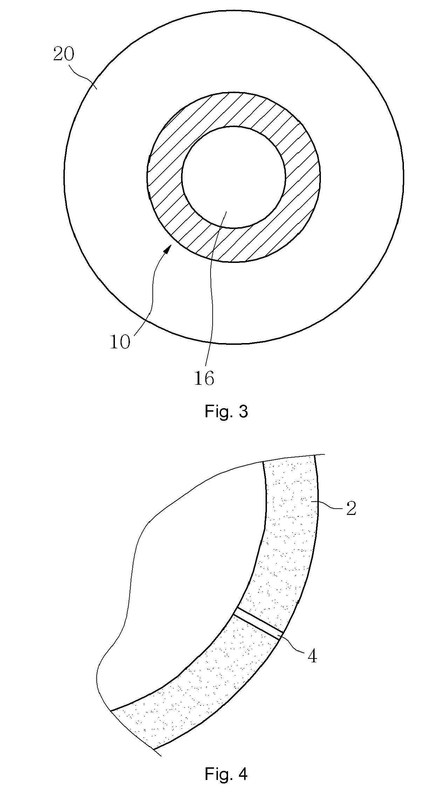

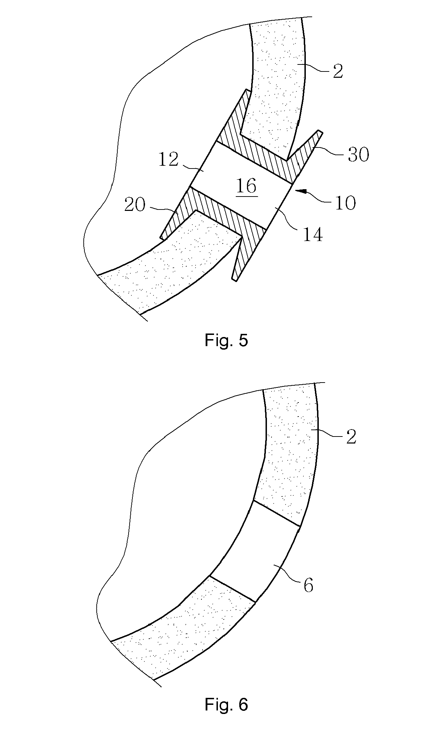

A drainage tube according to a first exemplary embodiment of the present invention is illustrated in FIGS. 1 to 6.

As illustrated in FIGS. 1 to 6, the drainage tube according to the first exemplary embodiment of the present invention includes a conduit member 10 which has a circular tube shape having a predetermined length, and a first wing member 20 and a second wing member 30 which are disposed at both sides of the conduit member 10, respectively.

The conduit member 10 has a first end 12 and a second end 14 which define both open ends of the conduit member 10, respectively, and a drainage passage 16 which is disposed in the conduit member 10 between the first end 12 and the second end 14. The drainage passage 16 spatially connects the first end 12 and the second end 14.

The first end 12 of the conduit member 10 is inserted into a diseased part 2 of the Bartholin's duct abscess through an incision 4 created by incising the diseased part 2, such that the pus or the like flows into the drainage passage 16 from the diseased part 2 through the first end 12 while the incision 4 that is a surgical site heals, and the pus or the like flowing into the drainage passage 16 is drained through the second end 14.

The first wing member 20 is provided on an outer circumference at a side of the first end 12 of the conduit member 10 in an outer circumferential direction and has a circular rim-shaped structure. The first wing member 20 is inserted into the diseased part 2 together with the first end 12 of the conduit member 10, thereby preventing the conduit member 10 inserted into the diseased part 2 from being separated from the diseased part 2.

The second wing member 30 is provided on an outer circumference at a side of the second end 14 of the conduit member 10 in an outer circumferential direction and has a circular rim-shaped structure. That is, the second wing member 30 has the same or similar shape as the first wing member 20. The second wing member 30 restricts the depth to which the conduit member 10 is inserted into the diseased part 2, and as a result, the second end 14 of the conduit member 10 inserted into the diseased part 2 is maintained to be exposed to the outside of the diseased part 2.

The conduit member 10, the first wing member 20, and the second wing member 30 may be formed integrally. In addition, the conduit member 10, the first wing member 20, and the second wing member 30 may be made of medical grade silicone or synthetic resin which has predetermined elasticity.

A surgical procedure for treatment of the Bartholin's duct abscess, which uses the drainage tube according to the first exemplary embodiment of the present invention, may be carried out as described below.

First, an outer wall of the diseased part 2 is incised to create the incision 4 through which the drainage tube is inserted into the diseased part 2.

Next, the first wing member 20, together with the first end 12 of the conduit member 10, is inserted into the diseased part 2 through the incision 4.

Here, a surgeon may more easily insert the first wing member 20 into the diseased part 2 because the first wing member 20 has predetermined elasticity. As an example, the shape of the first wing member 20 is naturally deformed while the first wing member 20 is being inserted into the diseased part 2, and as a result, the first wing member 20 may easily enter the diseased part 2. As another example, the surgeon may insert the first wing member 20 into the diseased part 2 while intentionally and appropriately deforming the shape of the first wing member 20.

In addition, because of a function of the second wing member 30 that catches the drainage tube, it is possible to prevent the drainage tube from being excessively inserted into the diseased part 2, thereby preventing even the second end 14 of the conduit member 10 from being inserted into the diseased part 2. In addition, because of a function of the first wing member 20 that catches the drainage tube, it is possible to prevent the drainage tube inserted into the diseased part 2 from being separated from the diseased part 2.

Next, the above state is maintained until the incision 4 that is the surgical site heals, and when the surgical site heals and a drainage opening 6, which corresponds to an outer circumference of the conduit member 10, is created in the surgical site, the inserted drainage tube is removed from the surgical site.

Here, during a period of time for which the surgical site heals, the pus or the like from the abscess is drained through the conduit member 10. Further, the conduit member 10 basically prevents the incision 4 from being closed overall, thereby assuredly creating the drainage opening 6.

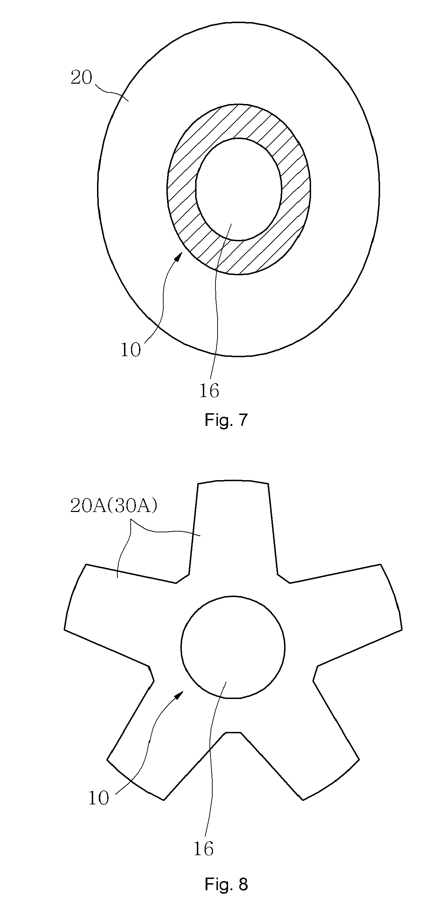

FIG. 7 is a transverse cross-sectional view illustrating a drainage tube according to a second exemplary embodiment of the present invention. As illustrated in FIG. 7, in the case of the drainage tube according to the second exemplary embodiment of the present invention, transverse cross sections of the conduit member 10 and the first and second wing members 20 and 30 may have an elliptical shape instead of a circular shape.

FIG. 8 is a front view illustrating a drainage tube according to a third exemplary embodiment of the present invention. As illustrated in FIG. 8, in the case of the drainage tube according to the second exemplary embodiment of the present invention, first and second wing members 20A and 30A may be configured as a plurality of separate wings that is disposed to be spaced apart from each other at intervals in the outer circumferential direction of the conduit member 10.

While the present invention has been described above, the present invention is not limited by the exemplary embodiments disclosed in the present specification and the accompanying drawings, and may be variously modified by those skilled in the art without departing from the technical spirit of the present invention.

For example, while it has been described that the first end 12 of the conduit member 10 is inserted into the diseased part 2 and the second end 14 is exposed to the outside of the diseased part 2, the second end 14 may be inserted into the diseased part 2.

## Claims

1. A drainage tube comprising:
a conduit member which has a first end, a second end, and a drainage passage disposed between the first end and the second end, the first end being inserted into a diseased part of a Bartholin's duct abscess through an incision created by incising the diseased part;
a first wing member which protrudes from an outer circumference of the conduit member at a side of the first end and prevents the conduit member inserted into the diseased part from being separated; and
a second wing member which protrudes from the outer circumference of the conduit member at a side of the second end and restricts the depth to which the conduit member is inserted into the diseased part.

2. The drainage tube of claim 1, wherein the first wing member and the second wing member are provided in an outer circumferential direction of the conduit member and have a rim-shaped structure.

3. The drainage tube of claim 1 or 2, wherein at least the first wing member of the first and second wing members is made of a material having predetermined elasticity.

4. The drainage tube of claim 1, wherein the conduit member, the first wing member, and the second wing member are formed integrally.

5. A drainage tube comprising:
a conduit member which has a first end, a second end, and a drainage passage disposed between the first end and the second end, the first end being inserted into a diseased part of a Bartholin's duct abscess through an incision created by incising the diseased part; and
a wing member which protrudes from an outer circumference of the conduit member at a side of the first end and prevents the conduit member inserted into the diseased part from being separated.

6. A drainage tube comprising:
a conduit member which has a first end, a second end, and a drainage passage disposed between the first end and the second end, the first end being inserted into a diseased part of a Bartholin's duct abscess through an incision created by incising the diseased part; and
a wing member which protrudes from an outer circumference of the conduit member at a side of the second end and restricts the depth to which the conduit member is inserted into the diseased part.
